# EUROPEAN PATENT SPECIFICATION

(11) **EP 1 099 446 B1**
(45) Date of publication and mention of the grant of the patent: **13.06.2007**
(21) Application number: 00309908.2
(22) Date of filing: 08.11.2000
(51) Int. Cl.: A61K 45/06, A61P 25/00

(54) **Combination for treating depression and anxiety containing a CNS-penetrant NK-1 receptor antagonist and an antidepressant or anxiolytic agent**
Einen ZNS-penetrierenden NK-1-Rezeptorantagonisten und ein angstlösendes oder antidepressives Mittel enthaltende Zusammensetzung zur Behandlung von Depression und Angst
Composition pour le traitement de la dépression et de l'anxieté contenant un antagoniste du récepteur NK-1 et un agent antidépressant ou anxiolitique

(30) Priority: 10.11.1999 US 164692 P
(43) Date of publication of application: 16.05.2001
(73) Proprietor: Pfizer Products Inc., Groton, Connecticut 06340 (US)
(72) Inventor: Sobolov-Jaynes, Susan Beth, Pfizer Global R&D, Groton, Connecticut 06340 (US)
(74) Representative: Ruddock, Keith Stephen

(56) References cited:
- WO-A-96/24353
- WO-A-98/15277

## Description

### Background of the Invention

The present invention relates to a method of treating depression or anxiety in a mammal, including a human, by administering to the mammal a CNS-penetrant NK-1 receptor antagonist (e.g., a substance P receptor antagonist) in combination with an antidepressant or an anxiolytic agent. It also relates to pharmaceutical compositions containing a pharmaceutically acceptable carrier, a CNS-penetrant NK-1 receptor antagonist and an anxiolytic agent or antidepressant.

Major depression is characterized by feelings of intense sadness and despair, mental slowing and loss of concentration, pessimistic worry, agitation, and self-deprecation. Physical changes also occur, especially in severe or "melancholic" depression. These include insomnia or hypersomnia, anorexia and weight loss (or sometimes overeating), decreased energy and libido, and disruption of normal circadian rhythms of activity, body temperature, and many endocrine functions.

Treatment regimens commonly include the use of tricyclic antidepressants, monoamine oxidase inhibitors, some psychotropic drugs, lithium carbonate, and electroconvulsive therapy (ECT) (see R. J. Baldessarini in Goodman & Gilman's The Pharmacological Basis of Therapeutics, 9th Edition, Chapter 19, McGraw-Hill, 1996 for a review). More recently, new classes of antidepressant drugs are being developed including selective serotonin re-uptake inhibitors (SSRIs), specific monoamine re-uptake inhibitors and 5-HTIA receptor agonists, antagonists and partial agonists.

Anxiety is an emotional condition characterized by feelings such as apprehension and fear accompanied by physical symptoms such as tachycardia, increased respiration, sweating and tremor. It is a normal emotion but when it is severe and disabling it becomes pathological.

Anxiety disorders are generally treated using benzodiazepine sedative-antianxiety agents. Potent benzodiazepines are effective in panic disorder as well as in generalized anxiety disorder, however, the risks associated with drug dependency may limit their long-term use. 5-HT_{IA} receptor partial agonists also have useful anxiolytic and other psychotropic activity, and less likelihood of sedation and dependence (see R. J. Baldessarini in Goodman & Gilman's Tite Pharmacological Basis of Therapeutics, 9th Edition, Chapter 18, McGraw-Hill, 1996 for a review).

### Summary Of The Invention

The present invention relates to a pharmaceutical composition for the treatment of anxiety or depression comprising: (a) a compound that exhibits activity as an antianxiety (i.e., anxiolytic) agent or an antidepressant, or a pharmaceutically acceptable salt thereof; (b) a CNS-penetrant NK-1 receptor antagonist or pharmaceutically acceptable salt thereof; and (c) a pharmaceutically acceptable carrier; wherein the active agents "a" and "b" above are present in amounts that render the composition effective in treating, respectively, anxiety or depression.

This invention also relates to a method of treating anxiety or depression in a mammal, comprising administering to said mammal, respectively, an anxiolytic or antidepressant effective amount of a pharmaceutical composition comprising: (a) a compound that exhibits activity as, respectively, an anxiolytic agent or an antidepressant, or a pharmaceutically acceptable salt thereof; (b) a CNS-penetrant NK-1 receptor antagonist or pharmaceutically acceptable salt thereof; and (c) a pharmaceutically acceptable carrier; wherein the active agents "a" and "b" above are present in amounts that render the composition effective in treating, respectively, anxiety or depression.

This invention also relates to a method of treating anxiety or depression in a mammal, comprising administering to said mammal: (a) a compound that exhibits activity as, respectively, an anxiolytic agent or an antidepressant, or a pharmaceutically acceptable salt thereof; and (b) a CNS-penetrant NK-1 receptor antagonist or pharmaceutically acceptable salt thereof; wherein the active agents "a" and "b" above are present in amounts that render the combination of the two agents effective in treating, respectively, anxiety or depression.

It will be appreciated that when using a combination of the present invention, referred to immediately above, both the CNS-penetrant NK-1 receptor antagonist and the antidepressant or anti-anxiety agent will be administered to a patient within a reasonable period of time. The compounds may be in the same pharmaceutically acceptable carrier and therefore administered simultaneously. They may be in separate pharmaceutical carriers such as conventional oral dosage forms that are taken simultaneously. The term combination, as used above, also refers to the case where the compounds are provided in separate dosage forms and are administered sequentially. Therefore, by way of example, the antidepressant or anxiolytic agent may be administered as a tablet and then, within a reasonable period of time, the CNS-penetrant NK-1 receptor antagonist may be administered either as an oral dosage form such as a tablet or a fast-dissolving oral dosage form. By a "fast dissolving oral formulation" is meant, an oral delivery form which when placed on the tongue of a patient, dissolves within about seconds.

The compositions of the present invention that contain an NK-1 receptor antagonist and an antidepressant are useful for the treatment of depression. As used herein, the term "depression" includes depressive disorders, for example, single episodic or recurrent major depressive disorders, and dysthymic disorders, depressive neurosis, and neurotic depression; melancholic depression including anorexia, weight loss, insomnia and early morning waking, and psychomotor retardation; atypical depression (or reactive depression) including increased appetite, hypersomnia, psychomotor agitation or irritability, anxiety and phobias, seasonal affective disorder, or bipolar disorders or manic depression, for example, bipolar I disorder, bipolar II disorder and cyclothymic disorder.

Other mood disorders encompassed within the term "depression" include dysthymic disorder with early or late onset and with or without atypical features; dementia of the Alzheimer's type, with early or late onset, with depressed mood; vascular dementia with depressed mood, disorders induced by alcohol, amphetamines, cocaine, hallucinogens, inhalants, opioids, phencyclidine, sedatives, hypnotics, anxiolytics and other substances; schizoaffective disorder of the depressed type; and adjustment disorder with depressed mood.

The compositions of the present invention that contain an NK-1 receptor antagonist and an anxiolytic agent are useful for the treatment of anxiety. As used herein, the term "anxiety" includes anxiety disorders, such as panic disorder with or without agoraphobia, agoraphobia without history of panic disorder, specific phobias, for example, specific animal phobias, social phobias, obsessive-compulsive disorder, stress disorders including post-traumatic stress disorder and acute stress disorder, and generalized anxiety disorders.

"Generalized anxiety" is typically defined as an extended period (e.g. at least six months) of excessive anxiety or worry with symptoms on most days of that period. The anxiety and worry is difficult to control and may be accompanied by restlessness, being easily fatigued, difficulty concentrating, irritability, muscle tension, and disturbed sleep.

"Panic disorder" is defined as the presence of recurrent panic attacks followed by at least one month of persistent concern about having another panic attack. A "panic attack" is a discrete period in which there is a sudden onset of intense apprehension, fearfulness or terror. During a panic attack, the individual may experience a variety of symptoms including palpitations, sweating, trembling, shortness of breath, chest pain, nausea and dizziness. Panic disorder may occur with or without agoraphobia.

"Phobias" includes agoraphobia, specific phobias and social phobias. "Agoraphobia" is characterized by an anxiety about being in places or situations from which escape might be difficult or embarrassing or in which help may not be available in the event of a panic attack. Agoraphobia may occur without history of a panic attack. A "specific phobia" is characterized by clinically significant anxiety provoked by feared object or situation. Specific phobias include the following subtypes: animal type, cued by animals or insects; natural environment type, cued by objects in the natural environment, for example storms, heights or water; blood-injection-injury type, cued by the sight of blood or an injury or by seeing or receiving an injection or other invasive medical procedure; situational type, cued by a specific situation such as public transportation, tunnels, bridges, elevators, flying, driving or enclosed spaces; and other type where fear is cued by other stimuli. Specific phobias may also be referred to as simple phobias. A "social phobia" is characterized by clinically significant anxiety provoked by exposure to certain types of social or performance circumstances. Social phobia may also be referred to as social anxiety disorder.

Other anxiety disorders encompassed within the term "anxiety" include anxiety disorders induced by alcohol, amphetamines, caffeine, cannabis, cocaine, hallucinogens, inhalants, phencyclidine, sedatives, hypnotics, anxiolytics and other substances, and adjustment disorders with anxiety or with mixed anxiety and depression.

Anxiety may be present with or without other disorders such as depression in mixed anxiety and depressive disorders. The compositions of the present invention are therefore useful in the treatment of anxiety with or without accompanying depression.

The compositions of the present invention are especially useful for the treatment of depression or anxiety where the use of an antidepressant or anxiolytic agent, respectively, is generally prescribed. By the use of a combination of a CNS-penetrant NK-1 receptor antagonist and an antidepressant or anxiolytic agent in accordance with the present invention, it is possible to treat depression and/or anxiety in patients for whom conventional antidepressant or antianxiety therapy might not be wholly successful or where dependence upon the antidepressant or antianxiety therapy is prevalent.

Suitable classes of antidepressant agents that may be used in the present invention include selective serotonin re-uptake inhibitors (SSRIs).

Suitable selective serotonin re-uptake inhibitors that may be used in the present invention include: fluoxetine, fluvoxamine, paroxetine and sertraline, and pharmaceutically acceptable salts thereof.

Examples of specific NK-1 receptor antagonists that can be used in the methods and pharmaceutical compositions of this invention are the following compounds and their pharmaceutically acceptable salts:
2-{3-[(2-Benzhydryl-1-aza-bicyclo[2.2.2]oct-3-ylamino)-methyl]-4-methbxy-phenyl}-2-methyl-propan-1-ol;
(2S,3S)-N-(5-isopropyl-2-methoxyphenyl)methyl-2-diphenymethyl-1-azabicyclo[2.2.2]octan-3-amine;
(2S,3S)-N-(5-tert-bulyl-2-methoxyphenyl)methyl-2-diphenylmethyl-1-azabicyclo[2.2.2]octane-3-amine;
(2S,3S)-N-(5-ethyl-2-methoxyphenyl)methyl-2-diphenylmethyl-1-azabicyclo[2.2.2]-octan-3-amine: and
(2S,3S)-N-(5-n-propyl-2-methoxyphenyl)methyl-2-diphenylmethyl-1-azabicyclo[2.2.2]octane-3-amine.

The terms "anxiolytic effective amount" and "antianxiety effective amount", as used herein, refer to an amount that is effective in treating anxiety.

The term "antidepressant effective amount", as used herein, refers to an amount that is effective in treating depression.

The term "treating" refers to, and includes, reversing, alleviating, inhibiting the progress of, or preventing a disease, disorder or condition, or one or more symptoms thereof; and "treatment" and "therapeutically" refer to the act of treating, as defined above.

The pharmaceutical compositions and uses of this invention comprise, or comprise administering NK-1 receptor antagonists which may have chiral centers and therefore exist in different enantiomeric forms. This invention includes uses and pharmaceutical compositions, as described above, wherein the NK-1 receptor antagonists that are employed are optical isomers, tautomers or stereoisomers of the compounds that are defined above, or mixtures thereof.

This present invention also relates to pharmaceutical compositions and methods comprising, or comprising administering, pharmaceutically acceptable acid addition salts of NK-1 receptor antagonists and of antidepressant and anxiolytic agents. The possible acids which are used to prepare the pharmaceutically acceptable acid addition salts of the basic active agents employed in the methods and pharmaceutical compositions of this invention are those which form non-toxic acid addition salts, *i.e.,* salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i.e*., 1, 1'-methylene-bis-(2-hydroxy-3- naphthoate)] salts.

This invention also relates to pharmaceutical compositions and methods comprising, or comprising administering, pharmaceutically acceptable base addition salts of NK-1 receptor antagonists and of antidepressant and anxiolytic agents. The chemical bases that may be used as reagents to prepare pharmaceutically acceptable base salts of the acidic active agents that are employed in this invention are those that form non-toxic base salts with such compounds. Such non-toxic base salts include, but are not limited to those derived from such pharmacologically acceptable cations such as alkali metal cations (*e.g*., potassium and sodium) and alkaline earth metal cations (*e.g.*, calcium and magnesium), ammonium or water-soluble amine addition salts such as N-methylglucamine (meglumine), and the lower alkanol ammonium and other base salts of pharmaceutically acceptable organic amines.

The subject invention also relates to pharmaceutical compositions and methods of treatment that employ isotopically-labeled compounds that are identical to the recited NK-1 receptor antagonists, but for the fact that one or more atoms are replaced by an atom having an atomic mass or mass number different from the atomic mass or mass number usually found in nature. Examples of isotopes that can be incorporated into the NK-1 receptor antagonists that are employed in the pharmaceutical compositions and methods of the present invention include isotopes of hydrogen, carbon, nitrogen, oxygen, phosphorous, fluorine and chlorine, such as ²H, ³H, ¹³C, ¹⁴C, ¹⁵N, ¹⁶O, ¹⁷O, ³¹P, ³²P, ³⁵S, ¹⁸F, and ³⁶Cl, respectively. The NK-1 receptor antagonists employed in the pharmaceutical compositions and uses of the present invention, prodrugs thereof, and pharmaceutical acceptable salts of said compounds or of said prodrugs which contain the aforementioned isotopes and/or other isotopes are within the scope of this invention. Certain isotopically-labeled NK-1 receptor antagonists, for example, those into which radioactive isotopes such as ³H and ¹⁴C are incorporated, are useful in drug and/or substrate tissue distribution assays. Tritiated. *i.e.*, ³H, and carbon-14, *i.e.,* ¹⁴C, isotopes are particularly preferred for their ease of preparation and detectability. Further, substitution with heavier isotopes such as deuterium, *i.e.,* ²H. can afford certain therapeutic advantages resulting from greater metabolic stability, for example increased *in vivo* half-life or reduced dosage requirements and, hence, may be preferred in some circumstances.

### Detailed Description of the Invention

This invention relates both to methods of treating anxiety or depression in which the NK-1 receptor antagonist and the anxiolytic or antidepressant agent, or pharmaceutically acceptable salts of the same, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, the emetogen and the severity of the condition. Generally the NK-1 receptor antagonist will be administered to an adult human in an amount ranging from about 0.05 to about 1500 mg per day, in single or divided doses, preferably from about 5 to about 200 mg/day. The compounds may be administered on a regimen of up to 6 times per day, preferably 1 to 4 times per day, especially 2 times per day and most especially once daily. A suitable dosage level for the antidepressant agent is about 0.5 to 1500 mg per day, preferably about 2.5 to 1000 mg per day, and especially about 2.5 to 500 mg per day. The compounds may be administered on a regimen of up to 6 time per day, preferably 1 to 4 times per day, especially 2 time per day and most especially once daily. A suitable dosage level for the anxiolytic agent is about 0.5 to 1500 mg per day, preferably about 2.5 to 1000 mg per day, and especially about 2.5 to 500 mg per day. The compounds may be administered on a regimen of up to 6 times per day, preferably 1 to 4 times per day, especially 2 times per day and most especially once daily. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The NK-1 receptor antagonists, their pharmaceutically acceptable salts, and the antidepressant and anxiolytic agents and their pharmaceutically acceptable salts that are employed in the pharmaceutical compositions and uses of this invention are hereinafter also referred to as "therapeutic agents". The therapeutic agents can be administered via either the oral or parenteral route. Compositions containing both an NK-1 receptor antagonist and an anxiolytic or antidepressant agent, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i.e.,* they may be combined with various pharmaceutical acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic agents of this invention, when administered separately (*i.e.,* not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably com, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tableting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the NK-1 receptor antagonist and the anxiolytic or antidepressant agent may be formulated in a single pharmaceutical composition or alternatively in individual pharmaceutical compositions for simultaneous, separate or sequential use in accordance with the present invention.

Preferably the compositions according to the present invention, which contain both an NK-1 receptor antagonist and an anxiolytic agent or an antidepressant, as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, *e.g.,* conventional tableting ingredients such as corn starch, lactose, sucrose, sorbitol, talc, stearic acid, magnesium stearate, dicalcium phosphate or gums, and other pharmaceutical diluents, *e.g.,* water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from 0.05 to about 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration of an NK-1 receptor antagonist or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g*., Tween^{™} 20, 40, 60, 80 or 85) and other sorbitans (*e.g*., Span^{™} 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid^{™}, Liposyn^{™}, Infonutrol^{™} , Lipofundin^{™} and Lipiphysan^{™}. The therapeutic agent may be either dissolved in a pre-mixed emulsion composition or alternatively it may be dissolved in an oil (*e.g*., soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable, aqueous or organic solvents or mixtures thereof, and powders. The liquid or solid compositions may contain suitable pharmaceutically acceptable excipients as set out above. Preferably, the compositions are administered by the oral or nasal respiratory route for local or systemic effect. Compositions in preferably sterile pharmaceutical acceptable solvents may be nebulised by use of inert gases. Nebulised solutions may be breathed directly from the nebulising device or the nebulising devise may be attached to a face mask, tent or intermittent positive pressure breathing machine. Solution, suspension, or powder compositions may be administered, preferably orally or nasally, from devices which deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising an NK-1 receptor antagonist and an antidepressant or anxiolytic agent, or pharmaceutically acceptable salts of the same, which process comprises bringing an NK-1 receptor antagonist and the antidepressant or anxiolytic agent (or the pharmaceutically acceptable salts of one or both of these therapeutic agents) into association with a pharmaceutically acceptable carrier or excipient.

It will be appreciated that the amount of the NK-1 receptor antagonist and the antidepressant or anxiolytic agent required for use in the treatment of depression or anxiety will vary not only with the particular compounds or compositions selected but also with the route of administration, the nature of the condition being treated, and the age and condition of the patient, and will ultimately be at the discretion of the patient's physician or pharmacist.

The activity of the compounds of the present invention, as substance P antagonists, is determined by their ability to inhibit the binding of substance P at its receptor sites in CHO-cells which reveal NK-1 receptor or IM-9 cells employing radioactive ligands. The substance P antagonist activity of the herein described piperidine compounds is evaluated using the standard assay procedure described by M. A. Cascieri et al., as reported in The Journal of Immunology, 133, 3260 (1984). This method essentially involves determining the concentration of the Individual compound required to reduce by 50% the amount of radiolabelled substance P ligands at their receptor sites In said isolated cow tissues or IM-9 cells, thereby affording characteristic IC₅₀ values for each compound tested. More specifically, inhibition of [³H]SP binding to human IM-9 cells by compounds is determined in assay buffer (50 mM Tris-HCl (ph 7.4), 1mM MnCl₂, 0.02% bovine serum albumin, bacitracin (40 µg/ml) leupeptin (4 µg/ml), chymostatin (2 µg/ml) and phosphoramidon (30 µg/ml). The reaction is initiated by the addition of cells to the assay buffer containing 0.56 nM [³H]SP and various concentrations of compounds (total volume; 0.5 ml) and allowed to incubate for 120 minutes at 4°C. Incubation is terminated by filtration onto GF/B filters (presoaked in 0.1% polyethylenimine for 2 hours). Nonspecific binding is defined as the radioactivity remaining in the presence of 1µM SP. The filters are placed into tubes and counted using a liquid scintillation counter.

When administered in combination, either as a single or as separate pharmaceutical composition(s), the CNS-penetrant NK-1 receptor antagonist and an antidepressant or anti-anxiety agent, are presented in a ratio which is consistent with the manifestation of the desired effect. In particular, the ratio by weight of the CNS-penetrant NK-1 receptor antagonist and the antidepressant or anxiolytic agent will suitably be between 0.001 to 1 and 1000 to 1, and especially between 0.01 to 1 and 100 to 1.

As used herein the term "patient" includes animals of economic importance such as bovine, ovine, and porcine animals, especially those that produce meat, as well as domestic animals (*e.g*. cats and dogs), sports animals (*e.g*. horses), zoo animals, and humans, the latter being preferred.

As used herein, the term "CNS-penetrant" refers to NK-1 receptor antagonists which are able to inhibit NK-1 receptor agonist-induced foot-tapping in the gerbil as hereinafter defined.

Essentially, hind foot-tapping in the gerbil induced by infusion of the NK-1 receptor agonist, GR73632 (d Ala[L-Pro⁹,Me-Leu¹⁰] -substance P(7-1 1)), under anaesthesia, directly into the central ventricles is inhibited when a CNS-penetrant NK-1 receptor antagonist is administered Intravenously immediately prior to GR73632 challenge, wherein hind foot-tapping over a period of five minutes following recovery from the anaesthesia is inhibited with an ID₅₀≤3mg/kg, and preferably with an ID₅₀≤1mg/kg.

In an alternative method, the NK-1 receptor antagonist is administered orally, I hour prior to GR73632 challenge, wherein the foot-tapping over a period of five minutes following recovery from anaesthesia is inhibited with an ID₅₀≤30mg/kg, and preferably with an ID₅₀≤10mg/kg.

CNS-penetrant NK-1 receptor antagonists of use in the present invention are also effective in the attenuation of separation-induced vocalizations by guinea-pig pups as hereinafter defined.

Essentially, a vocalization response in guinea-pig pups is induced by isolation from their mothers and littermates, which response is attenuated when a CNS-penetrant NK- I receptor antagonist is administered subcutaneously 30 minutes prior to isolation, wherein vocalizations during the first 15 minutes of isolation are attenuated with an ID₅₀≤20mg/kg, preferably with an ID₅₀≤10mg/kg, and especially with an ID₅₀≤5mg/kg.

In an alternative method, the NK-1 receptor antagonist is administered orally, 4 hours prior to isolation, wherein vocalizations during the first 15 minutes of isolation are attenuated with an ID₅₀≤20mg/kg, preferably with an ID₅₀≤10mg/kg, and especially with an ID₅₀≤5mg/kg.

The following examples illustrate pharmaceutical compositions according to the invention. The formulations may be prepared with separate active ingredients or with a combination of active ingredients in one composition. In such combined preparations, the ration of the CNS-penetrant NK-1 receptor antagonist and the antidepressant or anti-anxiety agent will depend upon the choice of active ingredients.

### EXAMPLE 1

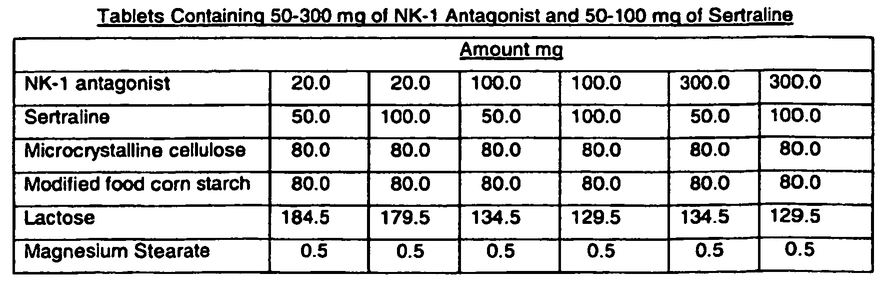

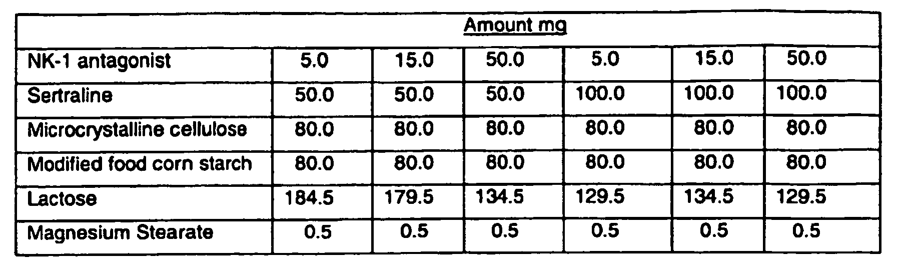

### EXAMPLE 2

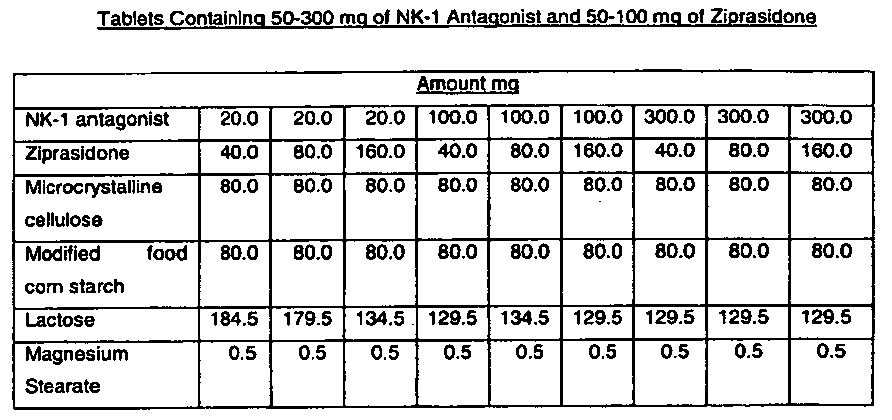

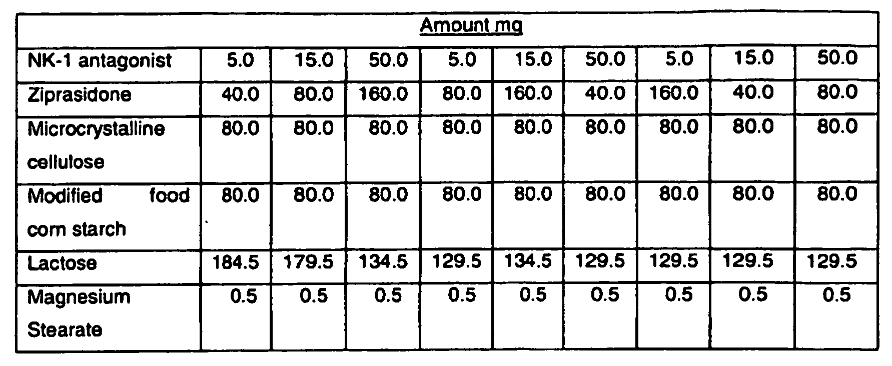

The active ingredients cellulose, lactose and a portion of the corn starch are mixed and granulated with 10% corn starch paste. The resulting granulation is sieved, dried and blended with the remainder of the corn starch and the magnesium stearate.

## Claims

1. A pharmaceutical composition comprising: (a) a compound that exhibits activity, as an anxiolytic agent or as an antidepressant, selected from fluoxetine, fluvoxamine, paroxetine and sertraline or a pharmaceutically acceptable salt thereof; (b) a CNS- penetrant NK-1 receptor antagonist selected from:
2-{3-[(2-Benzhydryl-1-aza-bicyclo[2.2.2]oct-3-ylamino)-methyl]-4-methoxyphenyl}-2-methyl-propan-1-ol;
2*S*,3*S*)-N-(5-isopropyl-2-methoxyphenyl)methyl-2-diphenylmethyl-1-azabicyclo[2.2.2]-octan-3-amine;
(2*S*,3*S*)-N-(5-tert-butyl-2-methoxyphenyl)methyl-2-diphenylmethyl-1-azabicyclo[2.2.2]-octan-3-amine;
(2*S*,3*S*)-N-(5-ethyl-2-methoxyphenyl)methyl-2-diphenylmethyl-1-azabicyclo[2-2.2]-octan-3-amine; and
(2*S*,3*S*)-N-(5-n-propyl-2-methoxyphenyl)methyl-2-diphenylmethyl-1-azabicyclo[2.2.2]-octan-3-amine;
or a pharmaceutically acceptable salt thereof; and (c) a pharmaceutically acceptable carrier or diluent.

2. The use of a compound that exhibits activity as an anxiolytic agent or as an antidepressant, selected from fluoxetine, fluvoxamine, paroxetine and sertraline or a pharmaceutically acceptable salt thereof in the preparation of a medicament, combined with a CNS-penetrant NK-1 receptor antagonist selected from:
2-{3-[(2-Benzhydryl-1-aza-bicyclo[2.2.2]oct-3-ylamino)-methyl]-4-methoxyphenyl}-2-methyl-propan-1-ol;
(2*S*,3*S*)-N-(5-isopropyl-2-methoxyphenyl)methyl-2-diphenylmethyl-1-azabicyclo[2.2.2]-octan-3-amine;
(2*S*,3*S*)-N-(5-tert-butyl-2-methoxyphenyl)methyl-2-diphenylmethyl-1-azabicyclo[2.2.2]-octan-3-amine;
(2*S,*3*S*)-N-(5-ethyl-2-methoxyphenyl)methyl-2-diphenylmethyl-1-azabicyclo[2.2.2]-octan-3-amine; and
(2*S*,3*S*)-N-(5-n-propyl-2-methoxyphenyl)methyl-2-diphenylmethyl-1-azabicyclo[2.2.2]-octan-3-amine;
or pharmaceutically acceptable salt thereof, for the treatment of anxiety or depression respectively.

3. Use according to claim 2. wherein the NK-1 receptor antagonist is administered in an amount ranging from about 5 mg per day to about 200 mg per day.

## Patentansprüche

1. Pharmazeutische Zusammensetzung, umfassend: (a) eine Verbindung, die Aktivität als ein anxiolytischer Wirkstoff oder als ein Antidepressivum aufweist, ausgewählt aus Fluoxetin, Fluvoxamin, Paroxetin und Sertralin oder einem pharmazeutisch verträglichen Salz davon; (b) einen ZNS-penetrierenden NK-1-Rezeptor-Antagonisten, ausgewählt aus:
2-{3-[(2-Benzhydryl-1-aza-bicyclo[2.2.2]oct-3-ylamino)methyl]-4-methoxy-phenyl}-2-methyl-propan-1-ol;
(2S,3S)-N-(5-Isopropyl-2-methoxyphenyl)methyl-2-diphenylmethyl-1-azabicyclo[2.2.2]-octan-3-amin;
(2S,3S)-N-(5-tert.-Butyl-2-methoxyphenyl)methyl-2-diphenylmethyl-1-azabicyclo[2.2.2]-octan-3-amin;
(2S,3S)-N-(5-Ethyl-2-methoxyphenyl)methyl-2-diphenylmethyl-1-azabicyclo[2.2.2]-octan-3-amin und
(2S,3S)-N-(5-n-Propyl-2-methoxyphenyl)methyl-2-diphenylmethyl-1-azabicyclo[2.2.2]-octan-3-amin
oder einem pharmazeutisch verträglichen Salz davon; und (c) ein(en) pharmazeutisch verträglichen (verträgliches.) Träger oder Verdünnungsmittel.

2. Verwendung einer Verbindung, die Aktivität als ein anxiolytischer Wirkstoff oder als ein Antidepressivum aufweist, ausgewählt aus Fluoxetin, Fluvoxamin, Paroxetin und Sertralin oder einem pharmazeutisch verträglichen Salz davon, bei der Herstellung eines Medikaments, kombiniert mit einem ZNS-penetrierenden NK-1-Rezeptor-Antagonisten, ausgewählt aus:
2-{3-[(2-Benzhydryl-1-aza-bicyclo[2.2.2]oct-3-ylamino)methyl]-4-methoxy-phenyl}-2-methyl-propan-1-ol;
(2S,3S)-N-(5-Isopropyl-2-methoxyphenyl)methyl-2-diphenylmethyl-1-azabicyclo[2.2.2]-octan-3-amin;
(2S,3S)-N-(5-tert.-Butyl-2-methoxyphenyl)methyl-2-diphenylmethyl-1-azabicyclo[2.2.2]-octan-3-amin;
(2S,3S)-N-(5-Ethyl-2-methoxyphenyl)methyl-2-diphenylmethyl-1-azabicyclo[2.2.2]-octan-3-amin und
(2S,3S)-N-(5-n-Propyl-2-methoxyphenyl)methyl-2-diphenylmethyl-1-azabicyclo[2.2.2]-octan-3-amin
oder einem pharmazeutisch verträglichen Salz davon, zur Behandlung von Angst bzw. Depression.

3. Verwendung nach Anspruch 2, wobei der NK-1-Rezeptor-Antagonist in einer Menge verabreicht wird, die von etwa 5 mg pro Tag bis etwa 200 mg pro Tag reicht.

## Revendications

1. Composition pharmaceutique comprenant : (a) un composé qui manifeste une activité en tant qu'agent anxiolytique ou en tant qu'antidépresseur, sélectionné parmi la fluoxétine, la fluvoxamine, la paroxétine et la sertraline ou un sel pharmaceutiquement acceptable correspondant ; (b) un antagoniste du récepteur NK-1 pénétrant dans le SNC, sélectionné parmi :
• le 2-{3-[(2-benzhydryl-1-aza-bicyclo[2.2.2]oct-3-ylamino)-méthyl]-4-méthoxy-phényl}-2-méthyl-propan-1-ol ;
• la (2*S*,3*S*)-N-(5-isopropyl-2-méthoxyphényl)méthyl-2-diphénylméthyl-1-azabicyclo[2.2.2]-octan-3-amine ;
• la (2*S*,3*S*)-N-(5-tert-butyl-2-méthoxyphényl)méthyl-2-diphénylméthyl-1-azabicyclo[2.2.2]-octan-3-amine ;
• la (2*S*,3*S*)-N-(5-éthyl-2-méthoxyphényl)méthyl-2-diphénylméthyl-1-azabicyclo[2.2.2]-octan-3-amine ; et
• la (2*S*,3*S*)-N-(5-n-propyl-2-méthoxyphényl)méthyl-2-diphénylméthyl-1-azabicyclo[2.2.2]-octan-3-amine ;
ou un sel pharmaceutiquement acceptable correspondant ; et (c) un support ou diluant pharmaceutiquement acceptable.

2. Utilisation, dans la préparation d'un médicament, d'un composé qui manifeste une activité en tant qu'agent anxiolytique ou en tant qu'antidépresseur, sélectionné parmi la fluoxétine, la fluvoxamine, la paroxétine et la sertraline ou un sel pharmaceutiquement acceptable correspondant, , combiné avec un antagoniste du récepteur NK-1 pénétrant dans le SNC, sélectionné parmi :
• le 2-{3-[(2-benzhydryl-1-aza-bicyclo[2.2.2]oct-3-ylamino)-méthyl]-4-méthoxy-phényl}-2-méthyl-propan-1-ol ;
• la (2*S*,3*S*)-N-(5-isopropyl-2-méthoxyphényl)méthyl-2-diphénylméthyl-1-azabicyclo[2.2.2]-octan-3-amine ;
• la (2*S*,3*S*)-N-(5-tert-butyl-2-méthoxyphényl)méthyl-2-diphénylméthyl-1-azabicyclo[2.2.2]-octan-3-amine ;
• la (2*S*,3*S*)-N-(5-éthyl-2-méthoxyphényl)méthyl-2-diphénylméthyl-1-azabicyclo[2.2.2]-octan-3-amine ; et
• la (2*S*,3*S*)-N-(5-n-propyl-2-méthoxyphényl)méthyl-2-diphénylméthyl-1-azabicyclo[2.2.2]-octan-3-amine ;
ou un sel pharmaceutiquement acceptable correspondant, pour le traitement, respectivement, de l'anxiété ou de la dépression.

3. Utilisation selon la revendication 2, dans laquelle l'antagoniste du récepteur NK-1 est administré en une quantité comprise dans la gamme allant d'environ 5 mg par jour à environ 200 mg par jour.
